# DEMANDE DE BREVET EUROPEEN

(11) **EP 2 749 296 A1**
(43) Date de publication de la demande: **02.07.2014**
(21) Numéro de dépôt: 13199789.2
(22) Date de dépôt: 30.12.2013
(51) Int. Cl.: A61L 9/12, B65B 63/08, B65D 75/58, B65D 43/20, B65B 3/04

(54) **Procédé d'emballage d'une matière chargée d'un produit actif et emballage utilisable pour la mise en oeuvre de ce procédé**

(30) Priorité: 31.12.2012 FR 1262996
(71) Demandeur: Roux, Philippe, 01150 Lagnieu (FR)
(72) Inventeur: Roux, Philippe, 01150 Lagnieu (FR)
(74) Mandataire: Jeannet, Olivier

(57) **Abrégé**

Ce procédé comprend les étapes consistant à :
- utiliser comme récipient une barquette (1) en matière synthétique, par exemple injectée ou thermoformée, comprenant un fond et une paroi périphérique formant un rebord périphérique ;
- couler directement ladite matière (2) dans cette barquette (1), laquelle sert de moule non réutilisable ;
- après solidification de ladite matière (2), fermer la barquette (1) avec un opercule (3) sous forme d'un film, fixé audit rebord périphérique, cet opercule (3) comprenant au moins deux parties amovibles (38, 39), distantes l'une de l'autre, séparables des parties adjacentes de l'opercule (3) et/ou dudit périphérique de manière à aménager, par leur retrait, aux moins deux ouvertures (4, 5) dans cet opercule, à savoir une ouverture (4) d'entrée d'air dans la barquette (1) et une ouverture (5) de diffusion du produit actif (22) contenu dans ladite matière (2).

## Description

### Domaine technique de l'invention.

La présente invention se situe dans le domaine du conditionnement, de l'utilisation, de la diffusion et du rejet de matières chargées d'un produit actif, utilisées par exemple pour des parfums, des désinfectants, des insecticides, des destructeurs d'odeurs, etc. Elle concerne en particulier un procédé d'emballage d'une matière chargée d'un produit actif, cette matière étant apte à passer d'un état liquide ou semi-liquide, c'est-à-dire plus ou moins pâteux, qu'elle a au moment de son versage dans un récipient de réception, à un état solide que cette matière acquiert une fois versée dans ce récipient. Elle concerne également un emballage utilisable pour la mise en oeuvre de ce procédé.

Les matières en question sont du type de celles qui sont contenues dans un emballage et qui, au moment de l'utilisation, sont exposées à l'air et diffusent le produit actif qu'elles contiennent dans cet air, sur une période prolongée.

Une matière telle que précitée est notamment une matière dite "gel polymère" bien connue pour les applications précitées ; il peut également s'agir d'une substance inerte minérale, notamment de plâtre.

### État de la technique antérieure.

De manière habituelle, la fabrication de gel polymère se fait dans des moules réutilisables dans lesquels on coule la préparation. Le gel, une fois polymérisé, est enlevé du moule puis est conditionné soit dans une filoche soit dans une boite en matière plastique munie d'ouvertures, réemballée dans un sac plastique. Pour utiliser le produit, l'utilisateur doit déchirer le sac plastique.

Ces emballages existants ont pour inconvénient d'induire le risque que l'utilisateur puisse accéder au gel et le toucher.

Pour empêcher que l'utilisateur puisse accéder au gel, ou autre substrat, et le toucher, il est connu par le document FR 2 328 612 A1 de prévoir une coque en deux parties fixées de manière permanente l'une à l'autre. La partie antérieure de cette coque comprend des trous, dont un, plus grand que les autres, permet le coulage du gel dans la coque, et dont les autres sont destinés à diffuser le parfum contenu dans le gel. Hors utilisation, un couvercle recouvrant les trous est appliqué sur ladite partie antérieure afin de clore ces trous et empêcher l'évaporation du parfum.

Cette structure d'emballage est considérée comme non optimale compte tenu de sa complexité de structure et de la complexité de la mise en oeuvre de son procédé de fabrication.

### Objectif de l'invention.

L'invention a pour objectif de remédier à l'inconvénient précité, en fournissant un procédé d'emballage d'une matière chargée d'un produit actif et un emballage utilisable pour la mise en oeuvre de ce procédé, ayant pour résultat que l'utilisateur ne puisse pas accéder facilement à la matière se trouvant dans l'emballage, ni toucher cette matière, sans affecter pour autant la libération du produit actif dont est chargée ladite matière, et ce, avec une structure d'emballage simple à fabriquer et à utiliser.

À cet effet, le procédé selon l'invention comprend les étapes consistant à :
- utiliser une barquette en matière synthétique, par exemple injectée ou thermoformée, comprenant un fond et une paroi périphérique formant un rebord périphérique ;
- couler directement ladite matière dans cette barquette, laquelle sert de moule non réutilisable ;
- après solidification de ladite matière, fermer la barquette avec un opercule sous forme d'un film, fixé audit rebord périphérique, cet opercule comprenant au moins deux parties amovibles, distantes l'une de l'autre, séparables des parties adjacentes de l'opercule et/ou dudit périphérique de manière à aménager, par leur retrait, aux moins deux ouvertures dans cet opercule, à savoir une ouverture d'entrée d'air dans la barquette et une ouverture de diffusion du produit actif contenu dans ladite matière.

Ainsi, l'utilisateur ôte ainsi lesdites parties amovibles de cet opercule pour aménager lesdites ouvertures dans l'opercule.

Le procédé conforme à l'invention permet d'atteindre les objectifs évoqués précédemment. En effet, le fait que l'opercule soit en plusieurs parties, permet à l'utilisateur d'ouvrir partiellement la barquette en deux emplacements distants, ce qui permet à l'air de circuler à l'intérieur d'elle. Cette circulation se fait naturellement, par convection, si la barquette est placée en position verticale.

Il sera compris que par « opercule », on entend un film ou une membrane d'épaisseur réduite, de quelques dixièmes de millimètres.

De préférence, le procédé comprend la réalisation de prédécoupes sur l'opercule, individualisant lesdites parties amovibles.

Ces prédécoupes permettent le retrait facile de ces parties amovibles.

Le procédé comprend de préférence les étapes consistant à :
- prévoir ledit opercule en matière plastique thermoscellable, et
- thermosceller cet opercule sur le rebord périphérique de la barquette.

L'invention a également pour objet un emballage utilisable pour la mise en oeuvre de ce procédé, incluant :
- une barquette qui comprend un fond et une paroi périphérique formant un rebord périphérique, et
- un opercule sous forme d'un film, fixé audit rebord périphérique de fermeture de cette barquette, comprenant au moins deux parties amovibles distantes l'une de l'autre, qui sont amovibles par rapport aux parties adjacentes de l'opercule et/ou par rapport audit rebord périphérique de manière à aménager, par leur retrait, aux moins deux ouvertures dans cet opercule.

De préférence, l'opercule comprend des prédécoupes individualisant lesdites parties amovibles.

De préférence, la barquette est surmontée d'une réglette coulissant sur elle, permettant d'obturer totalement ou partiellement au moins une ouverture de l'opercule aménagée par au moins une desdites parties amovibles.

Cette réglette permet de régler ainsi le degré de la diffusion du produit actif.

De préférence, l'opercule présente plusieurs prédécoupes dans une même zone, délimitant entre elles des portions amovibles d'opercule qui peuvent être retirées indépendamment les unes des autres. Ces portions amovibles d'opercule peuvent notamment être adjacentes les unes aux autres.

Ainsi, les dimensions de l'ouverture d'entrée d'air et/ou de l'ouverture de diffusion peuvent être réglées selon que telle ou telle portion d'opercule délimité par ces prédécoupes est retirée ou laissée en place.

D'autres caractéristiques et avantages de l'invention apparaissent à la lecture de la description d'un mode particulier de réalisation, fait ci-après référence aux dessins annexés, dans lesquels :

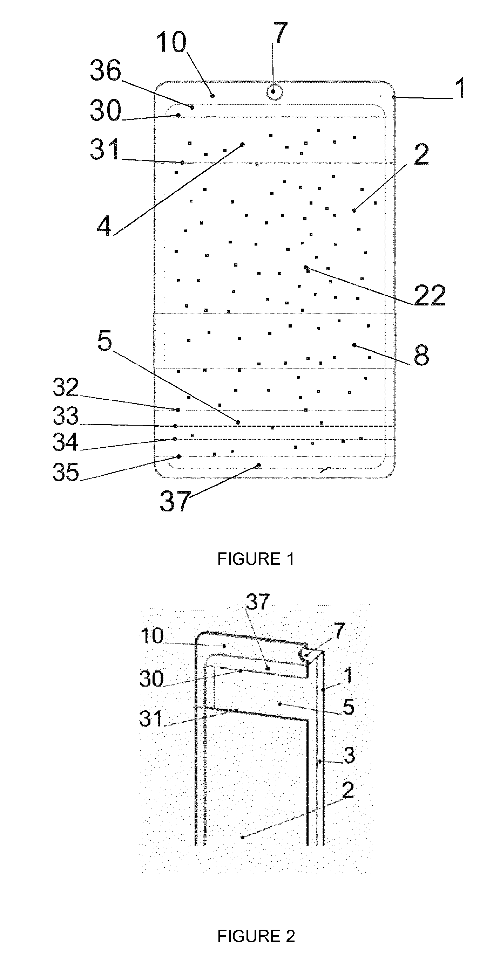

la figure 1 est une vue de dessus d'une barquette et de l'opercule permettant la mise en oeuvre du procédé conforme à l'invention ;
la figure 2 est une vue en coupe agrandie illustrant les différentes parties de l'opercule ;

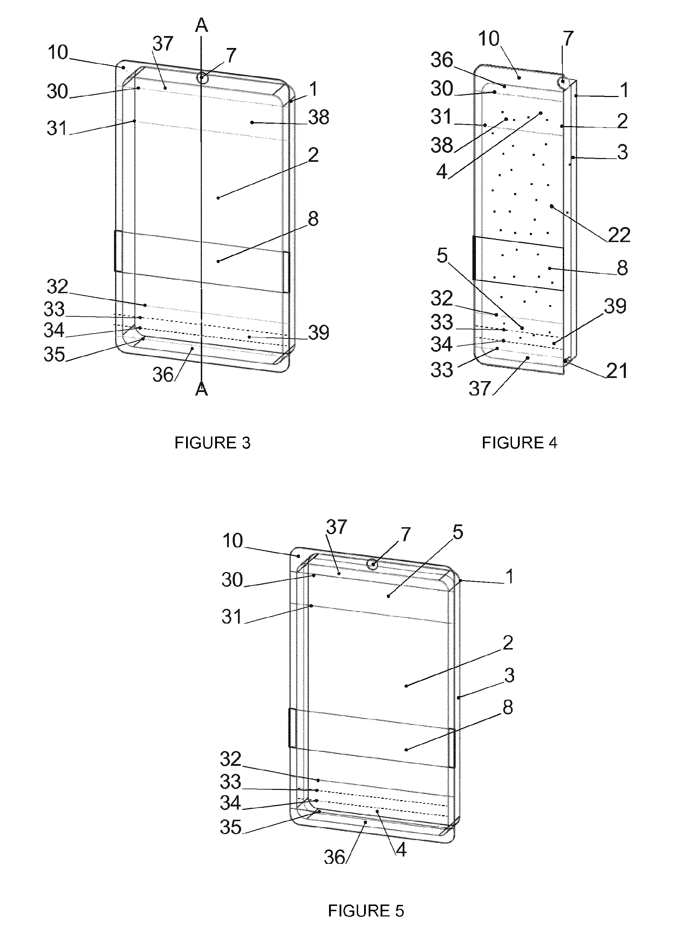

la figure 3 est une vue de la barquette en perspective ;
la figure 4 est une vue de la barquette en coupe suivant la ligne A-A, de la figure 3, illustrant les parties détachables de l'opercule ;
la figure 5 est une vue de la barquette en perspective, illustrant les ouvertures de l'opercule après enlèvement des parties détachables de cet opercule ;

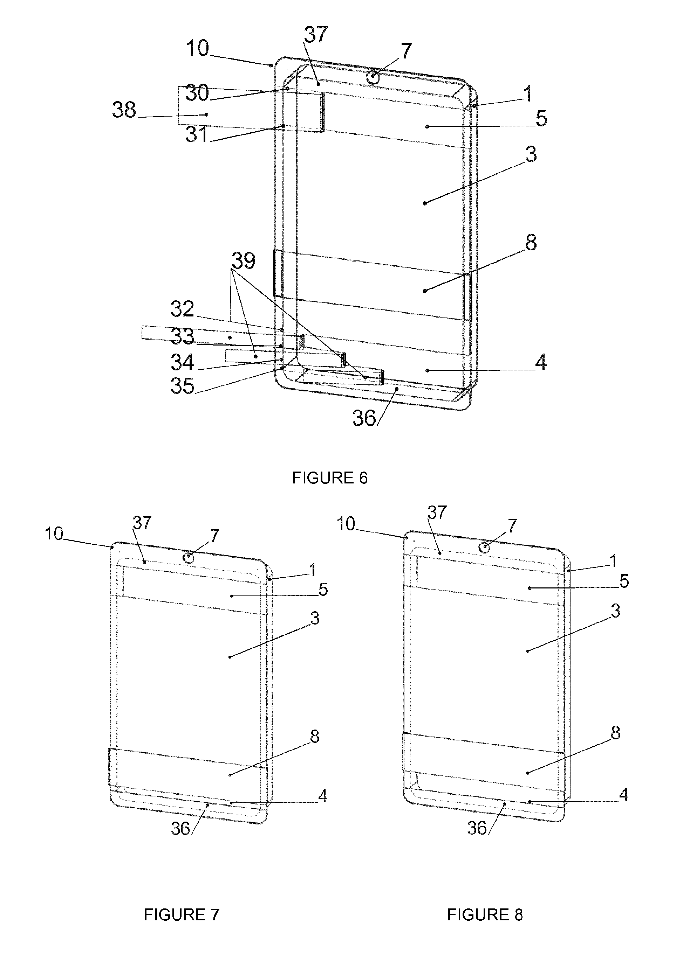

la figure 6 est une vue de la barquette en perspective, mettant en évidence les parties détachables de l'opercule ; et
les figures 7 et 8 sont des vues de la barquette en perspective, mettant en évidence la réglette coulissant sur le bord de la barquette.

### Description de l'invention.

Le repère (1) désigne la barquette.

Le repère (10) désigne le rebord extérieur périphérique s'étendant sur le pourtour de la barquette.

Le repère (2) désigne le gel polymère contenu dans la barquette.

Le repère (21) désigne les éventuels surplus des produits actifs.

Le repère (22) désigne les particules des produits actifs.

Le repère (3) désigne l'opercule, sous forme d'un film de quelques dixièmes de millimètres d'épaisseur.

Les repères (30) (31) (32) (33) (34) (35) désignent des prédécoupes aménagées dans l'opercule (3), transversalement à celui-ci.

Les repères (36) et (37) désignent les parties réservoirs de l'opercule.

Les repères (38) et (39) désignent les parties détachables de l'opercule.

Le repère (4) désigne l'ouverture d'entrée d'air.

Le repère (5) désigne l'ouverture de sortie d'air.

Le repère (6) désigne le volume d'air intérieur de la barquette.

Le repère (7) désigne le trou de fixation de la barquette pour la suspendre.

Le repère (8) désigne la réglette coulissante sur les bords d la barquette.

L'utilisation de la barquette (1) permet la mise en oeuvre d'un procédé d'emballage d'un gel polymère contenant un produit actif, comprenant les étapes suivantes :
- préparation du gel polymère (2) et des composants actifs inclus dans celui-ci ;
- versement de cette préparation dans la barquette (1), puis attente du temps nécessaire à la polymérisation de la préparation ;
- thermoscellage de l'opercule (3) sur le pourtour (10) de la barquette (1).

Les prédécoupes (30), (31), (32), (33), (34) et (35) réalisées sur l'opercule 3 permettent de retenir le gel polymère (2) ainsi que les éventuels surplus des produits actifs (21) qui pourraient suinter du gel polymère (2), afin de ne pas risquer de tacher les objets ou surfaces pouvant se trouver sous la barquette (1) après son installation.

L'utilisateur devra ôter les parties détachables (38) et (39) de l'opercule (3) puis suspendre la barquette (1) au moyen du trou de fixation (7).

Une convection naturelle permettra à l'air (6) d'entrer dans la baquette (2) par l'ouverture (4), de se charger par échange de particules produit actif (22) et de ressortir par l'ouverture (5), ceci afin de traiter l'air ambiant.

Il est visible sur les figures que les prédécoupes sont au nombre de deux en un premier emplacement de la barquette, en particulier situé à une extrémité longitudinale de celle-ci ; ces deux prédécoupes permettent d'individualiser la bande amovible 38. En un deuxième emplacement de la barquette, distant du premier emplacement, en particulier situé à l'autre extrémité longitudinale de la barquette, l'opercule comprend une pluralité de prédécoupes proches les unes des autres, aptes à être retirées indépendamment les unes des autres ; ces prédécoupes individualisent plusieurs bandes amovibles, dont le retrait ou non permet de régler la largeur de l'ouverture de diffusion du produit volatil présent dans le substrat contenu dans la barquette.

Une réglette (8) coulissant sur les bords de la barquette (1) permet à l'utilisateur de fermer plus ou moins l'arrivée ou la sortie d'air afin de régler le degré de la diffusion.

Quand le gel polymère (2) est consommé et n'a plus aucune action de diffusion, la barquette (1) peut être jetée avec les résidus de gel polymère (2) qu'elle contient.

## Revendications

1. Procédé d'emballage d'une matière (2) chargée d'un produit actif (22), cette matière (2) étant apte à passer d'un état liquide ou semi-liquide, qu'elle a au moment de son versage dans un récipient de réception, à un état solide que cette matière (2) acquiert une fois versée dans ce récipient, **caractérisé en ce qu'**il comprend les étapes consistant à :
- utiliser comme récipient une barquette (1) en matière synthétique, par exemple injectée ou thermoformée, comprenant un fond et une paroi périphérique formant un rebord périphérique ;
- couler directement ladite matière (2) dans cette barquette (1), laquelle sert de moule non réutilisable ;
- après solidification de ladite matière (2), fermer la barquette (1) avec un opercule (3) sous forme d'un film, fixé audit rebord périphérique, cet opercule (3) comprenant au moins deux parties amovibles (38, 39), distantes l'une de l'autre, séparables des parties adjacentes de l'opercule (3) et/ou dudit périphérique de manière à aménager, par leur retrait, aux moins deux ouvertures (4, 5) dans cet opercule, à savoir une ouverture (4) d'entrée d'air dans la barquette (1) et une ouverture (5) de diffusion du produit actif (22) contenu dans ladite matière (2).

2. Procédé d'emballage selon la revendication 1, **caractérisé en ce qu'**il comprend la réalisation de prédécoupes sur l'opercule (3), individualisant lesdites parties amovibles (38, 39).

3. Procédé d'emballage selon la revendication 1 ou la revendication 2, **caractérisé en ce qu'**il comprend les étapes consistant à :
- prévoir ledit opercule (3) en matière plastique thermoscellable, et
- thermosceller ledit opercule (3) sur le rebord périphérique de la barquette (1).

4. Emballage utilisable pour la mise en oeuvre du procédé selon l'une des revendications 1 à 3, **caractérisé en ce qu'**il inclut :
- une barquette (1) qui comprend un fond et une paroi périphérique formant un rebord périphérique, et
- un opercule (3) sous forme d'un film, fixé audit rebord périphérique de fermeture de cette barquette (1), comprenant au moins deux parties amovibles (38, 39) distantes l'une de l'autre, qui sont amovibles par rapport aux parties adjacentes de l'opercule (3) et/ou par rapport audit rebord périphérique de manière à aménager, par leur retrait, aux moins deux ouvertures (4, 5) dans cet opercule.

5. Emballage selon la revendication 4, **caractérisé en ce que** la barquette (1) est surmontée d'une réglette (8) coulissant sur elle, permettant d'obturer totalement ou partiellement ladite ouverture de diffusion (5) et régler ainsi le degré de la diffusion du produit actif (22).

6. Emballage selon la revendication 4 ou la revendication 5, **caractérisé en ce que** l'opercule (3) présente plusieurs prédécoupes (30), (31), (32), (33), (34), (35) dans une même zone, délimitant entre elles des portions amovibles d'opercule (3) qui peuvent être retirées indépendamment les unes des autres.

7. Emballage selon la revendication 6, **caractérisé en ce que** les portions amovibles d'opercule sont adjacentes les unes aux autres.
